# EUROPEAN PATENT SPECIFICATION

(11) **EP 2 450 053 B1**
(45) Date of publication and mention of the grant of the patent: **15.06.2016**
(21) Application number: 10190167.6
(22) Date of filing: 05.11.2010
(51) Int. Cl.: A61K 39/02, A61K 48/00, C07K 14/315, C07K 16/12, G01N 33/15

(54) **Novel antigen of enterococcal pathogens and use thereof as vaccine component for therapy and/or prophylaxis**
Neuartiges Antigen von Enterokokkenpathogenen und ihre Verwendung als Impfstoffkomponenten für Therapie und/oder Prophylaxe
Nouvel antigène de pathogènes entérocoques et leur utilisation en tant que composant de vaccin pour le traitement et/ou la prévention

(43) Date of publication of application: 09.05.2012
(73) Proprietor: Universitätsklinikum Freiburg, 79106 Freiburg (DE)
(72) Inventor: Hübner. Johannes Prof. Dr., 79115 Freiburg (DE); Grohmann, Elizabeth, 79112 Freiburg (DE); Kropec-Hübner, Andrea Dr., 79115 Freiburg (DE)
(74) Representative: Krauss, Jan

(56) References cited:
- DATABASE UniProt [Online] 1 October 2002 (2002-10-01), "SubName: Full=Putative uncharacterized protein;", XP002622147, retrieved from EBI accession no. UNIPROT:Q8L1C7 Database accession no. Q8L1C7 & THOMPSON J K ET AL: "Completed sequence of plasmid pIP501 and origin of spontaneous deletion derivatives.", July 2003 (2003-07), PLASMID, VOL. 50, NR. 1, PAGE(S) 28-35 ISSN: 0147-619X
- ABAJY MOHAMMAD Y ET AL: "A type IV-secretion-like system is required for conjugative DNA transport of broad-host-range plasmid pIP501 in gram-positive bacteria", JOURNAL OF BACTERIOLOGY, vol. 189, no. 6, March 2007 (2007-03), pages 2487-2496, XP002622148, ISSN: 0021-9193
- ROMERO-STEINER SANDRA ET AL: "Use of opsonophagocytosis for serological evaluation of pneurnococcal vaccines", CLINICAL AND VACCINE IMMUNOLOGY, vol. 13, no. 2, February 2006 (2006-02), pages 165-169, XP002622149, ISSN: 1556-6811
- DATABASE GENESEQ [Online] 29 May 2008 'Human guanylin sequence # 149.' Retrieved from EBI, accession no. GSP:AOG47833 Database accession no. AOG47833

### SEQUENCE LISTING

<110> Universitätsklinikum Freiburg
<120> Novel antigen of enterococcal pathogens and use thereof as vaccine component for therapy and/or prophylaxis
<130> FB23826
<160> 1
<170> PatentIn version 3.5
<210> 1
   <211> 322
   <212> PRT
   <213> Enterococcus faecium
<400> 1
   Met Ser Tyr Tyr Phe Glu Ile Arg Ile Ile Leu Pro Glu Glu Glu Asn
   1               5                   10                  15
   Gln Phe Leu Asn Arg Lys Leu Ser Lys Ser Glu Leu Ser Glu Val Thr
               20                  25                  30
   His Tyr Leu Gln Gln Lys Thr Ser Arg Gly Ile Pro Val Lys Phe Arg
           35                  40                  45
   Val Gly Ile Phe Arg Val Glu Asp Gln Thr Lys Ile Met Ser Val Thr
       50                  55                  60
   Leu Asn Thr Lys Asn Thr Lys Glu Thr Asp Val Ile Asn Leu Leu Leu
   65                  70                  75                  80
   Asn Arg Val Thr Asp Gln His Val Leu Val Tyr Leu Asn Glu Pro Thr
                   85                  90                  95
   Glu Pro Thr Leu Asn Thr Gln Glu Leu Asn Arg Gln Glu Leu Lys Thr
               100                 105                 110
   Ser Asn Glu Arg Gln Glu Ile Pro Gln Thr Glu Ile Pro Thr Glu Thr
           115                 120                 125
   Val Asn Glu Pro Ser Val Ile Lys Lys Ile Ser Lys Lys Asn Gln Ala
       130                 135                 140
   Lys Gln Thr Asn Ser Arg Lys Glu Ser Leu Ser Glu Ser Ile Thr Lys
   145                 150                 155                 160
   Lys Asn Val Pro Lys Ile His Leu Phe Ile Ser Ile Leu Thr Leu Phe
                   165                 170                 175
   Ile Val Leu Leu Ile Gly Ile Ser Val Ile Gln Gln Val Gln Leu Gln
               180                 185                 190
   Ser Val Lys Lys Glu Ser Glu Leu Leu Glu Glu Gln Ile Glu Arg Val
           195                 200                 205
   Lys Glu Thr Asp Ile Ser Gln Ser Lys Ile Asp Thr Phe Gly Arg Tyr
       210                 215                 220
   Phe Leu Thr Tyr Tyr Phe Ser Gln Glu Lys Asn Gln Glu Asn Tyr Gln
   225                 230                 235                 240
   Ser Ser Leu Arg Thr Tyr Val Ser Glu Lys Val Asp Ile Ser Asp Trp
                   245                 250                 255
   Lys Ala Leu Gly Lys Thr Leu Lys Ser Val Asn Tyr Tyr Gly Ser Glu
               260                 265                 270
   Gln Thr Lys Lys Gly Tyr Ser Val Glu Tyr Leu Leu Asn Val Ser Val
           275                 280                 285
   Asp Asn Arg Ser Lys Met Gln Lys Ile Thr Phe Glu Val Glu Pro Thr
       290                 295                 300
   Lys Asn Gly Phe Leu Val Thr Thr Gln Pro Lys Leu Thr Asp Phe Ser
   305                 310                 315                 320
   Phe Asn

## Description

The present invention relates to antigens, more particularly protein antigens of enterococcal pathogens which are useful as vaccine components for therapy and/or prophylaxis.

### Background of the invention

Enterococci are among the most important pathogens associated with infections in hospitalized patients. Especially the presence of multiple antibiotic resistance determinants in most clinically relevant isolates urges the development of alternative treatment and prevention strategies to combat these sometimes untreatable infections. Enterococci have developed specific mechanisms to acquire and transmit DNA horizontally, and these traits are responsible for numerous outbreaks in the hospital setting.

Bacterial conjugation is the most important means of gene delivery enabling adaptation of bacteria to changing environmental conditions including spread of antibiotic resistance genes, thereby generating multiply antibiotic resistant pathogens. Multiply resistant pathogens, such as *Enterococcus faecalis, Enterococcus faecium, Pseudomonas aeruginosa, Streptococcus pneumoniae,* and *Staphylococcus aureus* represent a serious threat to antibiotic treatment of hospitalized and immuno-suppressed patients (Grohmann 2006).

Bacterial conjugation systems are specialized types of type IV secretion systems (T4SS) dedicated to transport proteins (e.g., virulence factors, toxins) from bacterial pathogens to mammalian hosts. The conjugative T4SS have evolved to intercellularly transport DNA substrates in addition to proteins (Grohmann et al., 2003, Grohmann 2006).

pIP501 is a broad-host-range conjugative plasmid, originally isolated from the human pathogen *Streptococcus agalactiae* (Schaberg et al., 1982), the causative agent of sepsis and meningitis. It shows the broadest host range of conjugative plasmid transfer of any known mobile genetic element from Gram-positive bacteria, comprising virtually all studied Gram-positive bacteria including even filamentous multicellular *Streptomyces* and Gram-negative *Escherichia coli* (Kurenbach et al., 2003). Plasmid transfer requires a 15-kb plasmid-encoded transfer region comprising a complete T4SS encoding 15 transfer proteins. Several of these proteins have been characterized in some enzymatic detail, such as the T4SS ATPases Orf5 and Orf10 which deliver energy for the DNA transport process by the hydrolysis of ATP.

The Orf7 protein was demonstrated to be a lytic transglycosylase with cleavage activity on peptidoglycan isolated from Gram-positive *E. faecalis,* as well as from Gram-negative *E. coli.*

A complete protein-protein interaction map has been established for the 15 T4SS proteins by yeast two-hybrid assay and pull-down. On the basis of these data, the first molecular model for a T4SS from Gram-positive pathogens has been developed (Abajy et al., 2007).

The T4SS proteins can be divided into three families with respect to their function in the transfer process: the energy delivery family, the channel component family, and the putative surface proteins or adhesins (Alvarez-Martinez and Christie, 2009). Orf5 and Orf10 are members of the energy component family, Orfl5 is a putative member of the surface protein family, and the lytic transglycosylase Orf7 and Orfl3 belong to the channel component family.

For Orfl3, a scaffold function for the portion of the T4SS channel extending distally from the membrane, as shown for the T4SS subunits VirB8 and VirB10 of the *Agrobacterium tumefaciens* T-DNA transfer system, has been proposed (Alvarez-Martinez and Christie, 2009). Orf13 has one predicted transmembrane helix (Abajy 2007). In *E. faecalis* JH2-2 cell fractions Orfl3 was localized to the cell envelope using immunoblot with anti-Orf13 antibodies. No protein-protein interactions with other T4SS proteins could be identified.

US 2005-026218 describes isolated nucleic acids that encode polypeptides that interact with T4SS, in particular the *Rickettsia sibirica* rsib_orf.1266 polypeptide.

Since infections caused by enterococci are of globally increasing importance, considerable effort has been devoted to the development of new treatment strategies against these pathogens. The main protective defense mechanism of the human immune system against enterococci is phagocytosis, which may occur through direct recognition of certain enterococcal surface structures or through opsonization by antibodies and complement.

The opsonophagocytic assay has been used to simulate the immune response in vitro and to identify enterococcal virulence factors. However, only few antigens have been identified so far that may offer the potential of inducing a protective immune response, and therefore would be promising vaccine targets.

The present invention is defined in the claims.

According to a first aspect the invention discloses an isolated peptide for use according to claim 1. Also disclosed is a protein comprising an Orfl3 sequence according to SEQ ID No. 1, b) a homolog of Orf13 selected from the sequences according to SEQ ID No. 2 to 9, for use in the treatment of diseases.

Orf13 as isolated from the *Enterococcus faecalis* plasmid pIP501 has the following amino acid sequence according the GenBank Accession number AJ505823.1 (UNIPROT Q8L1C7):

In addition, Orf13-homologs have been identified in two *E. faecium* strains E1679 (catheter tip isolate) and on the *E. facium* plasmid pVEF3 (protein p51); on five *E. faecalis* strains: HIP11704 (unnamed plasmid), *E. faecalis* DS5 (unnamed plasmid), *E. faecalis* RE25 (plasmid pRE25, T4SS almost identical with pIP501), *E. faecalis* DS5 (plasmid pAMbeta1), and *E. faecalis* ADM24818, and in one *S. pyogenes* strain (plasmid pSM19035) as follows:

| Bacterial strain | Isolate/note/ref. | Acc. No. (as of November 1, 2010), SEQ ID No. |
|---|---|---|
| *E. faecium* | strain E1679; catheter tip isolate | ZP_06698913.1 SEQ ID No. 2 |
| *E. faecium* | plasmid pVEF3; protein p51 | YP_001974820.1, SEQ ID No. 3 |
| *E. faecalis* | HIP11704 | ZP_05569726.1 SEQ ID No. 4 |
| *E. faecalis* | DS5 | ZP_05563503.1 SEQ ID No. 5 |
| *E. faecalis* | RE25; pRE25, T4SS almost identical with pIP501 | YP_783921.1 SEQ ID No. 6 |
| *E. faecalis* | DS5; plasmid pAMbeta1 | YP_003305375.1 SEQ ID No. 7 |
| *E. faecalis* | ADM24818; Zhu et al., 2010 | ADM24818.1 SEQ ID No. 8 |
| *S. pyogenes* | plasmid pSM19035 | YP_232773.1 SEQ ID No. 9 |

Therefore, ORF 13 seems to be present in several pathogenic bacteria, such as, for example, E. *faecalis* and *E. faecium* strains, and thus is a promising vaccine target for active or passive immunotherapy of, for example, enterococci.

Since appropriate animal models for an effective analysis of the immunogenicity and protective efficacy towards bacterial virulence factors are missing, the opsonophagocytic assay is used as a surrogate to simulate the mammalian in vivo immune response in vitro, and to identify enterococcal virulence factors that can be developed into, for example, effective antibacterial vaccines (Markus Hufnagel, Steffi Koch, Andrea Kropec, Johannes Huebner: Opsonophagocytic assay as a potentially useful tool for assessing safety of enterococcal preparations. International Journal of Food Microbiology 88 (2003) 263- 267).

For Orfl3, a scaffold function for the portion of the T4SS channel extending distally from the membrane, as shown for the T4SS subunits VirB8 and VirB10 of the *Agrobacterium tumefaciens* T-DNA transfer system, has been proposed (Alvarez-Martinez and Christie, 2009). Orf13 has one predicted transmembrane helix (Abajy 2007). In *E. faecalis* JH2-2 cell fractions Orfl3 was localized to the cell envelope using immunoblot analysis with anti-Orf13 antibodies. Therefore, preferred is the peptide according to the present invention, wherein said peptide is a channel-forming component, and preferably has the activity to build portion of the T4SS channel.

Another aspect of the present invention then relates to an antibody or fragment thereof that specifically binds to any of the Orf13 peptides for use as a medicament in the context of the present invention. Preferred is an antibody or fragment thereof for use according to the present invention, wherein said antibody or fragment thereof is selected from a monoclonal antibody, an scFV fragment and a Fab-fragment. The antibodies can further be human or humanized antibodies or fragments. Methods to produce antibodies are known to the person of skill and described in the respective literature.

The present invention provides new therapeutic approaches in order to treat bacterial infections, such as infections caused by *Enterococcus faecalis, Enterococcus faecium, Pseudomonas aeruginosa, Streptococcus pneumoniae, Staphylococcus aureus* and *coagulase-negative staphylococci,* as well as strains of *Clostridia, Listeria or S. pyogenes.,* and in particular bacterial infections that are caused by strains showing resistance against multiple antibiotics, such as multiple resistant *Enterococcus faecalis, Enterococcus faecium, Pseudomonas aeruginosa, Streptococcus pneumoniae, Staphylococcus aureus* and *coagulase-negative staphylococci,* as well as strains of *Clostridia* or *Listeria.* Respective multiply resistant strains are also described in the literature. In this context, it is particular preferred that said homolog of Orfl3 is encoded by a plasmid isolated from such an antibiotic resistant strain of *E. faecium, E. faecalis,* or *S. pneumonia.*

Another aspect of the present invention then relates to a pharmaceutical composition for use according to claim 5 comprising the peptide according to the present invention (i.e. at least one of a protein comprising an Orfl3 sequence according to SEQ ID No. 1 or a homolog of Orfl3 selected from the sequences according to SEQ ID No. 2 to 9, or an antibody or antigenic fragment thereof that specifically binds to any of SEQ ID No. 1 or said homolog), and a pharmaceutically acceptable carrier, adjuvant and/or diluent.

Preferably, the pharmaceutical composition for use according to the present invention is a vaccine.

Thus, according to the present invention to another aspect, there are provided vaccine compositions comprising one or more peptides according to the present invention (i.e. at least one of a protein comprising an Orfl3 sequence according to SEQ ID No. 1, a homolog of Orfl3 selected from the sequences according to SEQ ID No. 2 to 9, or an antibody or antigenic fragment thereof that specifically binds to any of SEQ ID No. 1 or said homolog) in admixture with a pharmaceutically acceptable carrier diluent or adjuvant. Pharmaceutically acceptable carriers also include tetanus toxoid or diphteria toxoid. The pharmaceutical composition for use according to the invention can further comprise at least one cytokine.

Suitable adjuvants include oils i.e. Freund's complete or incomplete adjuvant; salts i.e. AlK(SO₄)₂, AlNa(SO₄)₂, AlNH₄(SO₄)₂, silica, kaolin, carbon polynucleotides, i.e. poly IC and poly AU. Preferred adjuvants include QuilA and Alhydrogel.

Another aspect of the present disclosure then relates to a pharmaceutical composition, wherein the peptide is covalently bonded or conjugated to an immunocarrier, such as capsular polysaccharides.

Vaccines of the invention may be administered parenterally by injection, rapid infusion, nasopharyngeal absorption, dermoabsorption, or bucal or oral. Said vaccine can also be formulated for administration via intramuscular, subcutaneous, or inhalation routes.

Another aspect of the present disclosure then relates to the peptide or the pharmaceutical composition according to the present invention for the prophylactic or therapeutic treatment of a disease or condition caused by a bacterium, and/or diseases and symptoms mediated by bacterial infections, such as, for example, enterococcal infection, urinary tract infections, bacteremia, bacterial endocarditis, peritonitis, wound or soft-tissue infections, pneumonia, and meningitis. Preferably, said bacterium is selected from enterococci, staphylococci or streptococci, such as, for example, *E. faecium, E. faecalis, S. aureus* or *S. pyogenes,* and in particular antibiotic-resistant strains thereof, and further preferably strains showing resistance against multiple antibiotics, such as multiple resistant *Enterococcus faecalis, Enterococcus faecium, Pseudomonas aeruginosa, Streptococcus pneumoniae,* and *Staphylococcus aureus.* Another aspect of the present disclosure then relates to use of the peptide or the pharmaceutical composition according to the present invention for the prophylactic or therapeutic treatment of a disease or condition as described here.

In a particularly preferred aspect the vaccines are administered to those individuals at risk of bacterial, and in particular enterococcal infection, such as infants, elderly and immunocompromised individuals.

As used in the present application, the term "individuals" include mammals. In a further embodiment, the mammal is human.

Vaccine compositions are preferably administered in a unit dosage form of about 0.001 to 100 Pg/kg (Peptide/body weight) and more preferably 0.01 to 10 Pg/kg and most preferably 0.1 to 1 Pg/kg 1 to 3 times with an interval of about 1 to 6 week intervals between immunizations.

Another aspect of the present disclosure then relates to a method for producing a pharmaceutical composition according to the present invention, preferably a vaccine, comprising admixing at least one of a protein comprising an Orfl3 sequence according to SEQ ID No. 1, a homolog of Orfl3 selected from the sequences according to SEQ ID No. 2 to 9, or an antibody or antigenic fragment thereof that specifically binds to any of SEQ ID No. 1 or said homolog with a pharmaceutically acceptable carrier diluent or adjuvant as described above.

Described is a method for therapeutic or prophylactic treatment of a disease or condition caused by a bacterium, and/or diseases and symptoms mediated by bacterial infections, such as, for example, bacterial infection, enterococcal infection, urinary tract infections, bacteremia, bacterial endocarditis, peritonitis, wound and soft tissue infections, and meningitis, or pneumonia in an individual, comprising administering to said individual a therapeutic or prophylactic amount of a pharmaceutical composition according to the present invention, preferably a vaccine. Preferably, said disease or condition is caused by a bacterium selected from enterococci, staphylococci or streptococci, such as, for example, *E. faecium, E. faecalis, S. aureus* or *S. pyogenes,* and in particular antibiotic-resistant strains thereof, and further preferably strains showing resistance against multiple antibiotics, such as multiple resistant *Enterococcus faecalis, Enterococcus faecium, Pseudomonas aeruginosa, Streptococcus pneumoniae,* and *Staphylococcus aureus or coagulase-negative staphylococci.*

Also described is a method for inducing an immune response against bacterial infection and in particular enterococcal infection, in a vertebrate, comprising administering to said vertebrate an immunologically effective amount of the pharmaceutical composition, and preferably a vaccine, according to the present disclosure.

Preferred is a method for the treatment and/or prophylaxis as described herein, wherein said method comprises administering intramuscularly, subcutaneously, or via inhalation to said vertebrate a therapeutically effective amount of a pharmaceutical composition, and preferably a vaccine, according to the present disclosure.

Further preferred is a method for the treatment and/or prophylaxis as described herein, wherein said method further comprises the administration of an active agent selected from additional antibiotics and/or preparations improving the activity of the immune system of the individual.

In the accompanying figure and the accompanying sequence listing,

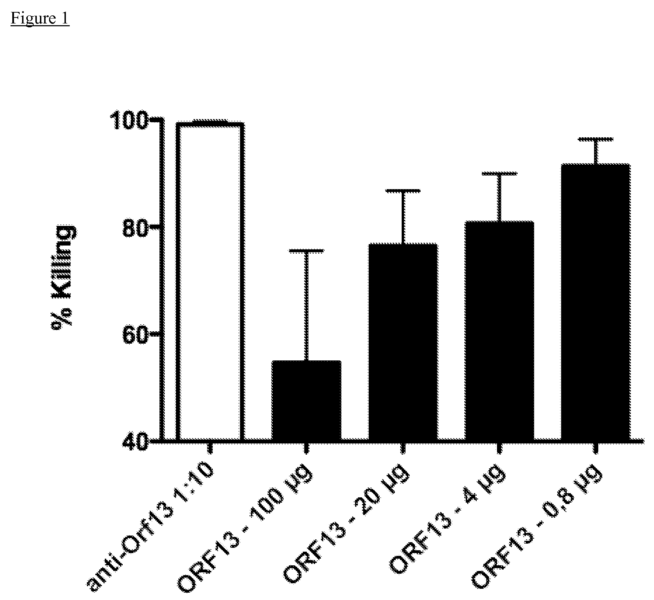

Figure 1 shows the result of the opsonophagocytic assay according to the Example below. White bar: unabsorbed serum raised against ORF13; black bars: anti-ORF13 serum absorbed with purified ORF13 protein.

SEQ ID No. 1: shows the amino acid sequence of Orfl3 as isolated from the *Enterococcus faecalis* plasmid pIP501 according to the GenBank Accession number AJ505823.1.

SEQ ID No. 2 to 9 show the amino acid sequences of homologs of Orf13 according to table 1. 2007), over-expressed and purified via affinity chromatography (Gössweiner-Mohr, N., Grohmann, E., and Keller, W., unpublished data). The purified protein was used to immunize a rabbit; similar prepared sera against ORF7 and ORF10 were used as controls.

Hyperimmune rabbit sera were examined by opsonophagocytic assay using leukocytes prepared from human volunteers and baby rabbit serum as complement source (Theilacker et al. Mol Microbiol 2009). Using undiluted serum there was 37.7% killing using the anti-ORF7 serum and no killing using anti-ORF8 and anti-ORF11 serum. Serum against ORF13, however, led to a 82.9% killing. Using *E. faecalis* type 9 as a control (since this strain is known to contain no T4SS) only 14% killing was observed using anti-ORF13 serum.

To confirm specificity a serum dilution of 1:10 was used and varying amounts of purified antigen were used as specific inhibitors. Preabsorption of the anti-ORF13 serum with 100 ug of purified protein led to a 45% inhibition, while lower amounts of inhibitory protein resulted in lower inhibition of killing (see Figure 1). These data confirm that ORF13 is the target of opsonic antibodies, and that absorption with this purified protein is able to remove the killing ability of the hyperimmune serum raised against ORF13.

In addition, Orfl3 has homologs in two *E. faecium* strains E1679 (catheter tip isolate) and on the *E. faecium* plasmid pVEF3 (protein p51), on five *E. faecalis* strains: HIP11704 (unnamed plasmid), *E. faecalis* DS5 (unnamed plasmid), *E. faecalis* RE25 (plasmid pRE25, T4SS almost identical with pIP501), *E. faecalis* DS5 (plasmid pAMbeta1) and in one *S. pyogenes* strain (plasmid pSM19035). Therefore, ORF13 seems to be present in several *E. faecalis* and *E. faecium* strains and is a promising vaccine target for active or passive immunotherapy of pathogenic bacteria, such as, for example, enterococci.

### Literature as cited

1. Abajy, M. Y. Molekularbiologische und biochemische Untersuchungen zum Typ IV Sekretion-ähnlichen System (T4SLS) des konjugativen Antibiotikaresistenzplasmids pIP501 in Enterococcus faecalis. (2007). PhD Thesis, TU Berlin, Berlin.
2. Abajy, M. Y., Kopec, J., Schiwon, K., Burzynski, M., Döring, M., Bohn, C., and Grohmann, E. (2007). A type IV-secretion-like system (T4SLS) is required for conjugative DNA transport of plasmid pIP501 with broad host range in Gram-positive bacteria J. Bacteriol. 189: 2487-2496.
3. Alvarez-Martinez, C. E., and Christie, P. J. (2009). Biological diversity of prokaryotic type IV secretion systems. Microbiol. Mol. Biol. Rev. 73: 775-808.
4. Grohmann, E., Muth, G., and Espinosa, M. (2003). Conjugative plasmid transfer in Gram-positive bacteria. Microbiol. Mol. Biol. Rev. 67: 277-301.
5. Grohmann, E. (2006). Mating cell-cell channels in conjugating bacteria. In Cell-Cell Channels (eds. Baluska, F., Volkmann, D., and Barlow, P.W.), Landes Biosciences, Georgetown, Texas, pp. 21-38. ISBN: 1-58706-065-5.
6. Kurenbach, B., Bohn, C., Prabhu, J., Abudukerim, M., Szewzyk, U., and Grohmann, E. (2003). Intergeneric transfer of the Enterococcus faecalis plasmid pIP501 to Escherichia coli and Streptomyces lividans and sequence analysis of its tra region. Plasmid 50: 86-93.
7. Schaberg, D.R., Clewell, D. B., and Glatzer, L. 1982. Conjugative transfer of R-plasmids from Streptococcus faecalis to Staphylococcus aureus. Antimicrob. Agents Chemother. 22:204-207.
8. Zhu, W., Murray, P.R., Huskins, W.C., Jernigan, J.A., McDonald, L.C., Clark, N.C., Anderson, K.F., McDougal, L.K., Hageman, J.C., Olsen-Rasmussen, M., Frace, M., Alangaden, G.J., Chenoweth, C., Zervos, M.J., Robinson-Dunn, B., Schreckenberger, P.C., Reller, L.B., Rudrik, J.T. and Patel, J.B. Dissemination of an Enterococcus Inc18-Like vanA Plasmid Associated with Vancomycin-Resistant Staphylococcus aureus, Antimicrob. Agents Chemother. 54 (10), 4314-4320 (2010).
9. Theilacker C, Sanchez-Carballo P, Toma I, Fabretti F, Sava I, Kropec A, Holst O, Huebner J. Glycolipids are involved in biofilm accumulation and prolonged bacteraemia in Enterococcus faecalis. Mol Microbiol. 2009 Feb;71(4):1055-69.

## Claims

1. An isolated peptide selected from
a) a protein comprising an Orf13 sequence according to SEQ ID No. 1, and
b) a homolog of Orf13 selected from the sequences according to SEQ ID No. 2 to 9,
for use as a medicament.

2. The peptide for use according to claim 1, wherein said peptide is a channel-forming component.

3. An antibody or fragment thereof that specifically binds to the peptide according to claim 1 for use as a medicament.

4. The antibody or fragment thereof for use according to claim 3, wherein said antibody or fragment thereof is selected from a monoclonal antibody, an scFV fragment and a Fab-fragment.

5. A pharmaceutical composition comprising the peptide for use according to claim 1, or the antibody or fragment thereof for use according to claim 3 or 4, and a pharmaceutically acceptable carrier, adjuvant and/or diluent.

6. The pharmaceutical composition for use according to claim 5, wherein said pharmaceutical composition is a vaccine.

7. The pharmaceutical composition for use according to claim 5 or 6, further comprising at least one cytokine.

8. The pharmaceutical composition for use according to claim 6, wherein said vaccine is formulated for administration via intramuscular, subcutaneous, or inhalation routes.

9. The peptide for use according to claim 1 or 2 or the pharmaceutical composition for use according to any of claims 5 to 8, wherein the disease or condition to be treated is caused by a bacterium, such as, for example, bacterial infection, enterococcal infection, urinary tract infections, bacteraemia, bacterial endocarditis, peritonitis, wound and soft tissue infections, and meningitis, or pneumonia.

10. The peptide or the pharmaceutical composition for use according to claim 9, wherein said bacterium is selected from enterococci, staphylococci or streptococci, such as, for example, *E*. *faecium, E. faecalis, S. aureus,* coagulase-negative staphylococci or S. *pyogenes,* and in particular antibiotic-resistant strains thereof.

## Patentansprüche

1. Isoliertes Peptid, ausgewählt aus
a) einem Protein umfassend eine Orf13 Sequenz nach SEQ ID No. 1, und
b) einem Homolog von Orf13 ausgewählt aus den Sequenzen nach SEQ ID No. 2 bis 9,
zur Verwendung als ein Medikament.

2. Peptid zur Verwendung nach Anspruch 1, wobei das Peptid eine Kanal-bildende Komponente ist.

3. Antikörper oder Fragment davon, der/das spezifisch an ein Peptid nach Anspruch 1 bindet, zur Verwendung als ein Medikament.

4. Antikörper oder Fragment davon zur Verwendung nach Anspruch 3, wobei der Antikörper oder das Fragment davon ausgewählt ist aus einem monoklonalen Antikörper, einem scFV Fragment und einem Fab-Fragment.

5. Pharmazeutische Zusammensetzung umfassend das Peptid zur Verwendung nach Anspruch 1, oder den Antikörper oder das Fragment davon zur Verwendung nach Anspruch 3 oder 4 und einen pharmazeutisch akzeptablen Träger, Adjuvans und/oder Verdünnungsmittel.

6. Pharmazeutische Zusammensetzung zur Verwendung nach Anspruch 5, wobei die Zusammensetzung ein Vakzin ist.

7. Pharmazeutische Zusammensetzung zur Verwendung nach Anspruch 5 oder 6, weiter umfassend mindestens ein Cytokin.

8. Pharmazeutische Zusammensetzung zur Verwendung nach Anspruch 6, wobei das Vakzin zur Verabreichung über intramuskuläre, subkutane oder Inhalations-Routen formuliert ist.

9. Peptid zur Verwendung nach Anspruch 1 oder 2, die pharmazeutische Zusammensetzung zur Verwendung nach einem der Ansprüche 5 bis 8, wobei die Erkrankung oder der zu behandelnde Zustand durch ein Bakterium verursacht wird, wie zum Beispiel bakterielle Infektion, Enterococcen-Infektion, Harnwegsinfektionen, Bakteriämie, bakterielle Endokarditis, Peritonitis, Infektionen von Wunden und weichem Gewebe und Meningitis, oder Pneumonie.

10. Peptid oder die pharmazeutische Zusammensetzung zur Verwendung nach Anspruch 9, wobei das Bakterium ausgewählt ist aus Enterococcen, Staphylococcen oder Streptococcen, wie zum Beispiel *E*. *faecium, E. faecalis, S. aureus,* Coagulase-negativen Staphylococcen oder *S. pyogenes,* und insbesondere Antibiotika-resistenten Stämmen davon.

## Revendications

1. Peptide isolé choisi parmi
a) une protéine comprenant une séquence d'Orf13 selon SEQ ID NO : 1, et
b) un homologue de l'Orf13 choisi parmi les séquences selon SEQ ID NO : 2 à 9,
pour une utilisation en tant que médicament.

2. Peptide pour une utilisation selon la revendication 1, où ledit peptide est un composant formant un canal.

3. Anticorps ou l'un de ses fragments qui se lie spécifiquement au peptide selon la revendication 1 pour une utilisation en tant que médicament.

4. Anticorps ou l'un de ses fragments pour une utilisation selon la revendication 3, où ledit anticorps ou l'un de ses fragments est choisi parmi un anticorps monoclonal, un fragment scFv et un fragment Fab.

5. Composition pharmaceutique comprenant le peptide pour une utilisation selon la revendication 1, ou l'anticorps ou l'un de ses fragments pour une utilisation selon la revendication 3 ou 4, et un support, un adjuvant et/ou un diluant pharmaceutiquement acceptable.

6. Composition pharmaceutique pour une utilisation selon la revendication 5, où ladite composition pharmaceutique est un vaccin.

7. Composition pharmaceutique pour une utilisation selon la revendication 5 ou 6, comprenant en outre au moins une cytokine.

8. Composition pharmaceutique pour une utilisation selon la revendication 6, où ledit vaccin est formulé pour une administration par les voies intramusculaire, sous-cutanée, ou par inhalation.

9. Peptide pour une utilisation selon la revendication 1 ou 2 ou composition pharmaceutique pour une utilisation selon l'une quelconque des revendications 5 à 8, où la maladie ou l'affection à traiter est provoquée par une bactérie, telle que, par exemple, une infection bactérienne, une infection entérococcique, des infections des voies urinaires, une bactériémie, une endocardite bactérienne, une péritonite, des infections de plaies et de tissus mous, et une méningite, ou une pneumonie.

10. Peptide ou composition pharmaceutique pour une utilisation selon la revendication 9, où ladite bactérie est choisie parmi des entérocoques, des staphylocoques ou des streptocoques, tels que, par exemple, *E. faecium, E. faecalis, S. aureus,* les staphylocoques coagulase négatifs ou *S. pyogenes,* et en particulier leurs souches résistantes aux antibiotiques.
